Europäisches Patentamt

European Patent Office

Office européen des brevets

(19)

(11) Publication number: **0 288 841 B1**

## EUROPEAN PATENT SPECIFICATION

(12)

(45) Date of publication of patent specification: **30.12.92**   (51) Int. Cl.5: **C07K 3/02**, A61K 37/64

(21) Application number: **88106017.2**

(22) Date of filing: **15.04.88**

(54) **Method of preparing highly purified alpha-1-proteinase inhibitor.**

(30) Priority: **27.04.87 US 42925**

(43) Date of publication of application:
**02.11.88 Bulletin 88/44**

(45) Publication of the grant of the patent:
**30.12.92 Bulletin 92/53**

(84) Designated Contracting States:
**AT BE CH DE ES FR GB GR IT LI NL SE**

(56) References cited:
**EP-A- 0 067 293**
**EP-A- 0 097 274**

**ANALYTICAL BIOCHEMISTRY, vol. 124, 1982, pages 364-371, Academic Press, Inc.; C.B. GLASER et al.: "The isolation of alpha-1-protease inhibitor by a unique procedure designed for industrial application"**

(73) Proprietor: **MILES INC.**
**1127 Myrtle Street**
**Elkhart Indiana 46514(US)**

(72) Inventor: **Shearer, Michael A.**
**4927 Silverado Drive**
**Suisun California 94585(US)**

(74) Representative: **Adrian, Albert, Dr. et al**
**c/o BAYER AG Konzernverwaltung RP Patentabteilung**
**W-5090 Leverkusen 1, Bayerwerk(DE)**

EP 0 288 841 B1

## Description

This invention relates to and has among its objects a method of preparing a high purity alpha-1-proteinase inhibitor (PI) concentrate from blood plasma, blood plasma fractions and concentrates. Further objects of the invention will be evident from the following description wherein parts and percentages are by weight unless otherwise specified.

Alpha-1-proteinase inhibitor is a glycoprotein having molecular weight of 54,000. The protein consists of a single polypeptide chain to which several oligosaccharide units are covalently bound. Human PI has a role in controlling tissue destruction by endogenous serine proteinases. A genetic deficiency of PI, which accounts for 90 % of the trypsin inhibitory capacity in blood plasma, has been shown to be associated with premature development of pulmonary emphysema. The degradation of elastin associated with emphysema probably results from a local imbalance of elastolytic enzymes and the naturally occurring tissue and plasma proteinase inhibitors. PI rapidly inhibits human pancreatic and leukocyte elastases (Biochem. Biophys. Res. Comm., Vol. 72, No. 1, pages 33-39, 1976; ibid., Vol. 88, No. 2, pages 346-350, 1979).

A number of methods employed to isolate PI from blood plasma are set forth in U.S. Patents 4,439,358, 4,379,087, and 4,656,254 and are incorporated herein by reference.

Pannell et al., Biochemistry, Vol. 13, pages 5439-5445, (1974), teaches a method of depleting albumin from plasma using Sepharose-Blue Dextran adsorption are then pooled and fractionated with solid ammonium sulfate.

Glaser et al., EP-A- 0.067.293 , teach the use of reducing agents, such as dithiothreitol to break disulfide bonds and precipitation by salting out with 50% $(NH_4)_2SO_4$, to remove unwanted proteins.

The invention described herein is a method for preparing a highly purified PI concentrate by depleting the albumin from a solution containing PI by adding a mild reducing agent sufficient to break the disulfide bonds in the albumin and heating the solution containing the PI and mild reducing agent to prevent the destabilized proteins from recombining.

In preferred embodiments, PI is separated from blood plasma or blood plasma fractions according to the Coan methods, U.S. Patents 4,379,087 and 4,439,358 and U.S. Serial No. 793,807. As well as the improvements set forth in U.S. Patent 4,656,254, Improved Methods of Preparing alpha-1-proteinase inhibitor. The reducing agent can be added to a solution of separated PI to break any protein disulfide bonds without reducing the PI, since PI does not have disulfide bonds. Reducing agents which can be utilized are dithiothreitol, dithioerythritol, 2-mercaptoethanol, mercaptoacetic acid, sodium borohydride and cysteine. The reducing agents are present in a concentration of about 2mM to 8mM. The concentration of reducing agent will vary depending on the type of solution containing PI and the choice of reducing agent. The amount of reducing agent should be sufficient to reduce the disulfide bonds without denaturing or damaging PI or any other protein to be recovered. The pH of the solution containing PI and the reducing agent can be adjusted to optimize the rate of reaction and also to stop the reaction. Heat denaturation of the reduced protein can be accomplished by raising the temperature to about 35°C or higher.

Suitable pH ranges are of about 7.9 to about 8.5 whereas the rates of reaction are about 5 to about 95 minutes.

Heat denaturation can also be accomplished in combination with a heat treating viral inactivation step. In U.S. Patent 4,470,968, there is disclosed a method of stabilizing coagulation Factors II, VIII, IX, and X against heat during pasteurization or heating at a temperature of about 60°-100°C by mixing with heat-stabilizing or pasteurization-stabilizing, amounts of a polyol and a source of citrate ions. Pasteurized compositions of these coagulation Factors were obtained by heating a mixture of unpasteurized protein composition, a polyol, and a source of citrate ions suspended or solubilized usually in an aqueous medium at a temperature and for a time sufficient to pasteurize the protein composition. Following pasteurization or heat treatment, the polyol and citrate ions are removed totally, or in part, as desired, from the protein composition by conventional techniques, and the pasteurized protein composition is processed according to conventional procedures for its ultimate therapeutic use. By way of illustration and not limitation, one may employ the method of U.S. Patent No. 4,470,968 (herein incorporated by reference) or any of the methods of the prior art described therein for heat inactivation.

One advantage of the invention is the availability of an essentially pure PI preparation that does not require the addition of ammonium sulfate to remove unwanted protein.

Another advantage of the invention is the incorporation of a step to remove unwanted protein with a heat inactivation step. The mild reducing agent can be added to the aqueous solution any time prior to the heat inactivation step and unwanted proteins removed when the pasteurized protein composition is processed according to conventional procedures for ultimate therapeutic use.

The reducing agent and denatured proteins can be removed by ultrafiltration and anion exchange methods without the addition of a salting out step.

Current methods for obtaining PI from blood plasma use as a starting material: Cohn Fraction IV and IV-1 and includes reworks of these fractions wherein other proteins are first removed, Cohn Effluent II + III and Cohn Effluent I and cryosupernatant solution. Blood plasma is fractionated according to the Cohn ethanol fractionation technique or its modifications [see for example, Cohn et al., J.Am. Chem. Soc., 68,459, (1946); Oncley et al., ibid., 71,541 (1949); U.S. Patent 2,390,074; "The Plasma Proteins", second edition, Vol. III, pages 548-550, Academic Press, New York, N.Y.] to give a Cohn fraction containing PI. This method for removing proteins containing disulfide bonds could have application in genetically engineered PI, in those situations where the solution containing PI, such a tissue culture medium or other eluates, also contained disulfide proteins, such as albumin.

PI was produced following the method outlined by Coan et al., Vox Sang. 48:333-342, 1985; U.S. Patents 4,379,087 and 4,439,358, and improvements set forth by Shearer et al., U.S. Patent 4,656,254.

Dithiothreitol was added to Coan's purified column eluate at a concentration of 5mM, pH was adjusted to 8.0-8.2. The solution was allowed to mix for twenty minutes and the reaction was stopped by adjusting the pH to 6.5. The PI solution containing the mild reducing agent was heat treated at 60°C for 10 hours in the presence of sucrose and citrate as indicated by Coan and Mitra, Vox Sang. 46:142-148 (1984) to render the solution free of virus. The denatured proteins were removed by applying the heat-treated solution to a DEAE Sepharose column equilibrated with 0.025M sodium phosphate at pH 6.5. The PI was removed by using a phosphate gradient to 0.1M at pH 6.5. The PI was found to be approximately 100% pure by Specific Activity (mg PI/$A_{280}$), CAE and HPLC. In contrast, the prior art method of Coan/Shearer provided a PI purity of about 60%.

The invention is related to a method of preparing highly purified alpha-1-proteinase inhibitor from a mixture of blood plasma proteins comprising

(a) adding a mild reducing agent to a mixture of plasma proteins containing alpha-1-proteinase inhibitor, said reducing agent being in a concentration sufficient to reduce disulfide bonds without reducing alpha-1-proteinase inhibitor;

(b) raising the temperature of the solution of step (a) to above 35°C; and

(c) recovering alpha-1-proteinase inhibitor from the solution.

The above steps being done in a manner that does not require the addition of ammonium sulfate. The above method of removing unwanted proteins from a mixture of plasma proteins containing alpha-1-proteinase inhibitor can be accomplished in modified form by the steps comprising

(a) adding a mild reducing agent to a mixture of plasma proteins containing alpha-1-proteinase inhibitor, said reducing agent being in a concentration sufficient to reduce disulfide bonds without reducing alpha-1-proteinase inhibitor;

(b) mixing the composition of Step (a) with a polyol selected from sucrose or reduced sugars and a source of citrate ions in an aqueous medium, said polyol and citrate ions being present in an amount sufficient to stabilize the protein during pasteurization;

(c) and heating the mixture at a temperature and for a time sufficient to pasteurize the composition.

Generally the amount of polyol and citrate ions in the mixture of Step (b) is about 20% to saturation of polyol. On a weight to volume basis and about 0.1-1.0 moles of citrate ion per liter of solution.

Heat denaturation can be accomplished independently of a viral inactivation step by raising the temperature of the solution containing PI and the mild reducing agent sufficient to reduce the disulfide bonds to a level above 35°C. The reducing agent can be removed by ultrafiltration. PI can be recovered from the heat treated solution by column chromatography using an appropriate anion exchange medium.

The mixture of the latter mentioned method is heated in step (c) at a temperature of about 60° - 100°C for a period of about 1 - 10 hours. The method further includes the Step of removing the polyol and citrate ions from the mixture of Step (c), for instance, by subjecting the mixture to diafiltration or by subjecting the mixture to dialysis or ion exchange chromatography. The concentration of citrate ions is about 0.3M to about 0.5M. A preferred embodiment is where the concentration of sucrose is, for instance, 37% (w/v) and the concentration of citrate ions is 0.38M.

The aqueous solution of plasma proteins containing alpha-1-proteinase inhibitor is preferably selected from the group of plasma fractions consisting of Cohn Fraction IV-1, Cohn Fraction IV, reworks of Cohn Fraction IV and IV-1, Cohn Effluent II + III, Cohn Effluent I, and cryosupernatant solution.

Especially the aqueous solution of plasma proteins containing alpha-1-proteinase inhibitor is selected from the group consisting of Cohn Fraction IV-1, Cohn Fraction IV, Cohn Effluent II + III and Cohn Effluent I.

Most preferred is a method wherein the aqueous solution of plasma proteins containing alpha-1-proteinase inhibitor is Cohn Fraction IV-1.

The invention is also related to a method for separating alpha-1-proteinase inhibitor from an aqueous solution of plasma proteins containing alpha-1-proteinase inhibitor which comprises the steps of:

(a) contacting an aqueous solution of one of the group of blood plasma and a blood fraction containing alpha-1-proteinase inhibitor with from about 8% to about 23% (w/v), based on volume of the aqueous solution, of a polycondensed polyalkylene glycol selected from polyethylene glycol and polypropylene glycol, at a temperature of from about 2°C to about 50°C for a period of about 0.2 - 24 hours to selectively precipitate unwanted proteins from the solution without precipitating alpha-1-proteinase inhibitor to obtain a mixture containing alpha-1-proteinase inhibitor free of unwanted proteins,

(b) recovering the mixture from step (a), and

(c) separating alpha-1-proteinase inhibitor from the mixture recovered in step (b), by column chromatography providing an eluate containing alpha-1-proteinase inhibitor,

which is characterised by the step of:

(i) adding a mild reducing agent to a mixture of plasma proteins containing alpha-1-proteinase inhibitor, said reducing agent being in a concentration sufficient to reduce disulfide bonds without reducing alpha-1-proteinase inhibitor;

(ii) raising the temperature of the solution of step (a) to above 35°C; and

(iii) recovering alpha-1-proteinase inhibitor from the solution.

The above steps being done in a manner that does not require the addition of ammonium sulfate.

The method according to the process at page 5 further includes the steps of

(i) providing for use in step (a) an aqueous solution of the one of the blood plasma and blood plasma fraction dissolved in from 20 to 100 volumes of buffer-solution per weight of the plasma or plasma fraction used, and

(ii) prior to use in step (a), adjusting one of the buffer solution and the resulting aqueous solution of the blood plasma and blood plasma fraction from step (1) to render the pH of the resulting solution at from 9.0 to 11.0.

Preferably in step (i) there is used from 20 to 50 volumes of buffer solution per weight of the one of the plasma and plasma fraction.

More preferred in step (i) there is used from 20 to 30 volumes of buffer solution per weight of the one of the plasma and plasma fraction and most preferred in step (i) there is used 24 volumes of buffer solution per weight of the one of the plasma and plasma fraction.

The pH of the one of the buffer solution and the resulting aqueous solution is adjusted to render the pH of the resulting solution from 9.0 to 10.5.

Preferably the pH of the one of the buffer solution and the resulting aqueous solution is adjusted to render the pH of the resulting solution from 9.0 to 10.5 and, more preferred the pH of the one of the buffer solution and the resulting aqueous solution is adjusted to render the pH of the resulting solution from 9.0 to 10.5.

## Claims

1. A method of preparing highly purified alpha-1-proteinase inhibitor from a mixture of blood plasma proteins comprising.

(a) adding a mild reducing agent to a mixture of plasma proteins containing albumin and alpha-1-proteinase inhibitor, said reducing agent being in a concentration sufficient to reduce disulfide bonds without reducing alpha-1-proteinase inhibitor;

(b) raising the temperature of the solution of step (a) to above 35°C; and

(c) recovering alpha-1-proteinase inhibitor from the solution,

the above steps being done in a manner that does not require. the addition of ammonium sulfate to remove unwanted protein.

2. The method of claim 1 wherein said mild reducing agent is selected from the group consisting of dithiothreitol, dithioerythritol, sodium borohydride, beta-mercaptoethanol, and cysteine.

3. The method of claim 2 wherein the concentration of said reducing agent is in the range of about 2mM to about 8mM.

4. The method of claim 3 wherein said reducing agent is 5mM dithiothreitol.

5. The method of claim 2 wherein said solution is mixed from about 5 to about 95 minutes at a pH range of about 7.9 to about 8.5.

6. The method of claim 5 wherein the temperature range is above 35°C.

7. The method of claim 4 wherein said solution is mixed for a period of about 20 minutes at a pH range of about 8.0 to about 8.2 at a temperature of about 25°C.

8. The method of claim 2 wherein said reaction is stopped by adjusting the pH to below 7.8.

9. The method of claim 8 wherein the pH is adjusted to about 6.5.

**10.** A method according to claim 1 including the further step of treating said aqueous solution of alpha-1-proteinase inhibitor obtained from step (c) to inactivate, and to reduce the infectivity of, infectious microorganisms so as to render the alpha-1-proteinase inhibitor non-infectious to such infectious microorganisms and thereby render the alpha-1-proteinase inhibitor useful for therapeutic and prophylactic purposes.

**11.** A method of preparing high purified alpha-1-proteinase inhibitor from a mixture of blood plasma proteins comprising

(a) adding a mild reducing agent to a mixture of plasma proteins containing alpha-1-proteinase inhibitor, said reducing agent being in a concentration sufficient to reduce disulfide bonds without reducing alpha-1-proteinase inhibitor;

(b) mixing the composition of Step (a) with a polyol selected from sucrose or reduced sugars and a source of citrate ions in an aqueous medium, said polyol and citrate ions being present in an amount sufficient to stabilize the protein during pasteurization;

(c) and heating the mixture at a temperature and for a time sufficient to pasteurize the composition.

**Patentansprüche**

**1.** Verfahren zur Herstellung von hochgereinigtem $\alpha$-1-Proteinaseinhibitoraus einer Mischung von Blutplasmaproteinen, umfassend:

(a) Zugabe eines milden Reduktionsmittels zu einer Mischung von Albumin- und $\alpha$-1-Proteinaseinhibitor-haltigen Plasmaproteinen, wobei das Reduktionsmittel in einer Konzentration vorliegt, die ausreichend ist, die Disulfidbindungen ohne Reduktion des $\alpha$-1-Proteinaseinhibitors zu reduzieren;

(b) Erhöhung der Temperatur der Lösung aus Schritt (a) auf ca. 35°C; und

(c) Gewinnung des $\alpha$-1-Proteinaseinhibitors aus der Lösung,

wobei die obigen Schritte in einer solchen Weise durchgeführt werden, so daß die Zugabe von Ammoniumsulfat zur Entfernung unerwünschten Proteins nicht erforderlich ist.

**2.** Verfahren nach Anspruch 1, worin das milde Reduktionsmittel ausgewählt ist aus Dithiothreitol, Dithioerythritol, Natriumborhydrid, $\beta$-Mercaptoethanol und Cystein.

**3.** Verfahren nach Anspruch 2, worin die Konzentration des Reduktionsmittels in einem Bereich von ca. 2 mM bis ca. 8 mM liegt.

**4.** Verfahren nach Anspruch 3, worin das Reduktionsmittel 5 mM des Dithiothreitol ist.

**5.** Verfahren nach Anspruch 2, worin die Lösung ca. 5 bis ca. 95 min in einem pH-Bereich von ca. 7,9 bis ca. 8,5 vermischt wird.

**6.** Verfahren nach Anspruch 5, worin der Temperaturbereich über 35°C liegt.

**7.** Verfahren nach Anspruch 4, worin die Lösung für einen Zeitraum von ca. 20 min in einem pH-Bereich von ca. 8,0 bis ca. 8,2 bei einer Temperatur von ca. 25°C vermischt wird.

**8.** Verfahren nach Anspruch 2, worin die Reaktion durch Einstellung des pHs auf unter 7,8 abgebrochen wird.

**9.** Verfahren nach Anspruch 8, worin der pH auf ca. 6,5 eingestellt wird.

**10.** Verfahren nach Anspruch 1, umfassend den zusätzlichen Schritt einer Behandlung der aus Schritt (c) erhaltenen wäßrgen a-1-Proteinaseinhibitor-haltigen Lösung, zur Inaktivierung und Infektiösitätsverminderung von infektiösen Mikroorganismen, um den $\alpha$-1-Proteinaseinhibitor nichtinfektiös bezüglich solcher infektiöser Mikroorganismen zu machen, und dadurch den $\alpha$-1-Proteinaseinhibitor für therapeutische und prophylaktische Verwendungen zugänglich zu machen.

**11.** Verfahren zu Herstellung von hochgereinigtem a-1-Proteinaseinhibitor aus einer Mischung aus Blutplasmaproteinen, umfassend:

(a) Zugabe eines milden Reduktionsmittels zu einer Mischung von $\alpha$-1-Proteinaseinhibitor-haltigen plasmaproteinen, wobei das Reduktionsmittel in einer Konzentration vorliegt, die ausreichend ist, die Disulfidbindungen ohne Reduktion des $\alpha$-1-Proteinaseinhibitors zu reduzieren;

(b) Vermischen der Zusammensetzung aus Schritt (a) mit einem Polyol, ausgewählt aus Saccharose und reduzierten Zuckern und einer Quelle von Citrationen in einem wäßrigen Medium, wobei das Polyol und die Citrationen in einer Menge vorliegen, die ausreichend ist, das Protein während der Pasteurisierung zu stabilisieren;

(c) und Erhitzen der Mischung auf eine Temperatur und für eine Zeit, die ausreichend für die Pasteurisierung der Zusammensetzung ist.

**Revendications**

1. Procédé de préparation d'un inhibiteur de l'alpha-1-protéinase très purifié à partir d'un mélange de protéines plasmatiques sanguines comprenant

(a) l'addition d'un agent faiblement réducteur à un mélange de protéines plasmatiques contenant de l'albumine et un inhibiteur de l'alpha-1-protéinase, ledit agent réducteur étant à une concentration suffisante pour réduire les liaisons disulfure sans réduire l'inhibiteur de l'alpha-1-protéinase ;

(b) l'élévation de la température de la solution de l'étape (a) à plus de 35°C ; et

(c) la récupération de l'inhibiteur de l'alpha-1-protéinase dans la solution,

les étapes ci-dessus étant opérées d'une manière qui ne nécessite pas l'addition de sulfate d'ammonium pour retirer la protéine indésirable.

2. Procédé selon la revendication 1, dans lequel ledit agent faiblement réducteur est sélectionné dans le groupe constitué par le dithiothréitol, le dithioérythritol, le borohydrure de sodium, le $\beta$-mercaptoéthanol et la cystéine.

3. Procédé selon la revendication 2, dans lequel la concentration dudit agent réducteur est comprise dans la plage d'environ 2 mM à environ 8 mM.

4. Procédé selon la revendication 3, dans lequel ledit agent réducteur est le dithiothréitol 5 mM.

5. Procédé selon la revendication 2, dans lequel ladite solution est mélangée pendant environ 5 à environ 95 minutes à un pH compris dans une plage d'environ 7,9 à environ 8,5.

6. Procédé selon la revendication 5, dans lequel la plage de températures est supérieure à 35°C.

7. Procédé selon la revendication 4, dans lequel ladite solution est mélangée pendant environ 20 minutes à un pH compris entre environ 8,0 et environ 8,2, à une température d'environ 25°C.

8. Procédé selon la revendication 2, dans lequel ladite réaction est arrêtée par ajustement du pH à un chiffre inférieur à 7,8.

9. Procédé selon la revendication 8, dans lequel le pH est ajusté à environ 6,5.

10. Procédé selon la revendication 1, comprenant l'étape supplémentaire de traitement de ladite solution aqueuse d'inhibiteur de l'alpha-1-protéinase obtenue à l'étape (c) pour inactiver et réduire le pouvoir infectant de micro-organismes infectieux de façon à rendre l'inhibiteur de l'alpha-1-protéinase non infectieux par de tels micro-organismes infectieux et à rendre ainsi l'inhibiteur de l'alpha-1-protéinase utile à des fins thérapeutiques et prophylactiques.

11. Procédé de préparation d'un inhibiteur de l'alpha-1-protéinase très purifié à partir d'un mélange de protéines plasmatiques sanguines comprenant

(a) l'addition d'un agent faiblement réducteur à un mélange de protéines plasmatiques contenant un inhibiteur de l'alpha-1-protéinase, ledit agent réducteur étant à une concentration suffisante pour réduire les liaisons disulfure sans réduire l'inhibiteur de l'alpha-1-protéinase ;

(b) le mélange de la composition de l'étape (a) avec un polyol sélectionné parmi le saccharose ou des sucres réduits et une source d'ions citrate dans un milieu aqueux, ledit polyol et lesdits ions citrate étant présents en une quantité suffisante pour stabiliser la protéine durant la pasteurisation ;

(c) et le chauffage du mélange à une température et pendant une durée suffisantes pour pasteuriser la composition.